# EUROPEAN PATENT APPLICATION

(11) **EP 3 108 804 A1**
(43) Date of publication of application: **28.12.2016**
(21) Application number: 14882582.1
(22) Date of filing: 11.09.2014
(51) Int. Cl.: A61B 1/06, G02B 23/26

(54) **OPTICAL FIBRE CONNECTOR DEVICE, AND ENDOSCOPE SYSTEM**

(30) Priority: 17.02.2014 JP 2014027801
(71) Applicant: Olympus Corporation, Tokyo 192-8507 (JP)
(72) Inventor: YOSHINO, Masahiro, Hachioji-shi, Tokyo 192-8507 (JP)
(74) Representative: Gunzelmann, Rainer
(86) International application number: PCT/JP2014/074084
(87) International publication number: WO 2015/122041

(57) **Abstract**

An optical fiber connector apparatus includes an optical receptacle provided with a first optical fiber configured to transmit light supplied from a light source section and a first lens disposed in proximity to an end face on an emission end side of the first optical fiber, an optical plug provided with a second lens and a second optical fiber configured to transmit light condensed by the second lens, a sleeve configured to hold the optical receptacle and the optical plug, and a displacement mechanism configured to displace a distance between the optical receptacle and the optical plug arranged opposite to each other.

## Description

### Technical Field

The present invention relates to an optical fiber connector apparatus and an endoscope system, and more particularly, to an optical fiber connector apparatus and an endoscope system having a configuration for making contactless connections between optical fibers.

### Background Art

Medical field endoscope systems are conventionally known which are configured to include, for example, an optical fiber on a light source side for transmitting laser light supplied from the light source and an optical fiber on an endoscope side for transmitting the laser light that has passed through the optical fiber on the light source side to a distal end side of the endoscope, and make contactless connections between the optical fibers. Japanese Patent Application Laid-Open Publication No. 2012-143414 discloses an endoscope apparatus similar to the aforementioned endoscope system.

More specifically, Japanese Patent Application Laid-Open Publication No. 2012-143414 discloses an endoscope apparatus including an endoscope and a light source apparatus connected to the endoscope, in which the light source apparatus is provided with a first optical fiber that transmits light emitted from the light source and a socket including a first collimator lens that expands a beam diameter of the light transmitted by the first optical fiber and collimates the light, and the endoscope is provided with a second collimator lens that condenses light transmitted from the first collimator lens and a connector including a second optical fiber that transmits the light condensed by the second collimator lens to a distal end side of the endoscope.

However, in the aforementioned endoscope system, for example, when the optical fiber on the light source side and the optical fiber on the endoscope side are constructed of a single mode fiber respectively, it is difficult to expand a luminous flux diameter of laser light by a certain factor or more of a core diameter of the laser light, which may result in a problem that a drastic fluctuation of coupling efficiency may occur relative to micro displacement in a gap of a joint for making contactless connections between optical fibers.

On the other hand, according to the configuration disclosed in Japanese Patent Application Laid-Open Publication No. 2012-143414, since both the first optical fiber and the second optical fiber are multimode fibers, the aforementioned problem is not taken into consideration. For that reason, in the configuration disclosed in Japanese Patent Application Laid-Open Publication No. 2012-143414 in which the first optical fiber and the second optical fiber are simply substituted by single mode fibers, brightness of an image captured by the endoscope easily fluctuates depending on a state of connection between the connector and the socket.

The present invention has been implemented in view of the aforementioned circumstances and it is an object of the present invention to provide an optical fiber connector apparatus and an endoscope system capable of preventing to the utmost, fluctuation of coupling efficiency occurring at a joint for making contactless connections between optical fibers.

### Disclosure of Invention

### Means for Solving the Problem

An optical fiber connector apparatus according to an aspect of the present invention is an optical fiber connector apparatus provided on a transmission path of light supplied from a light source section, including an optical receptacle provided with a first optical fiber configured to transmit light supplied from the light source section and a first lens disposed in proximity to an end face on an emission end side of the first optical fiber, an optical plug provided with a second lens and a second optical fiber configured to transmit light condensed by the second lens, a sleeve configured to hold the optical receptacle and the optical plug, and a displacement mechanism configured to displace an opposite distance between the optical receptacle and the optical plug.

An endoscope system according to an aspect of the present invention is an endoscope system configured by including an endoscope configured to include an insertion portion that can be inserted into a subject and a main unit connected to the endoscope and configured by including a light source section that supplies illuminating light for illuminating an object in the subject, the endoscope system including an optical receptacle provided in the main unit including a first optical fiber configured to transmit illuminating light supplied from the light source section and a first lens disposed in proximity to an end face on an emission end side of the first optical fiber, an optical plug provided at a proximal end portion of the insertion portion including a second lens and a second optical fiber configured to transmit illuminating light condensed by the second lens, a sleeve configured to hold the optical receptacle and the optical plug, a control section configured to detect a state of connection between the endoscope and the main unit, and a displacement mechanism configured to displace an opposite distance between the optical receptacle and the optical plug under control of the control section.

### Brief Description of the Drawings

Fig. 1 is a diagram illustrating a configuration of main parts of an endoscope system according to an embodiment;
Fig. 2 is a diagram for describing an example of a configuration of an optical plug according to the embodiment;
Fig. 3 is a diagram for describing an example of a configuration of an optical receptacle according to the embodiment;
Fig. 4 is a cross-sectional view along a line IV-IV of Fig. 3;
Fig. 5 is a diagram illustrating an example of a state of connection between the optical plug and the optical receptacle according to the embodiment;
Fig. 6 is a diagram illustrating an example different from that of Fig. 5 of the state of connection between the optical plug and the optical receptacle according to the embodiment;
Fig. 7 is a diagram illustrating an example different from those of Fig. 5 and Fig. 6 of the state of connection between the optical plug and the optical receptacle according to the embodiment;
Fig. 8 is a diagram for describing an example different from that of Fig. 2 of the configuration of the optical plug according to the embodiment; and
Fig. 9 is a diagram for describing an example different from that of Fig. 3 of the configuration of the optical receptacle according to the embodiment.

### Best Mode for Carrying Out the Invention

Hereinafter, an embodiment of the present invention will be described with reference to the accompanying drawings.

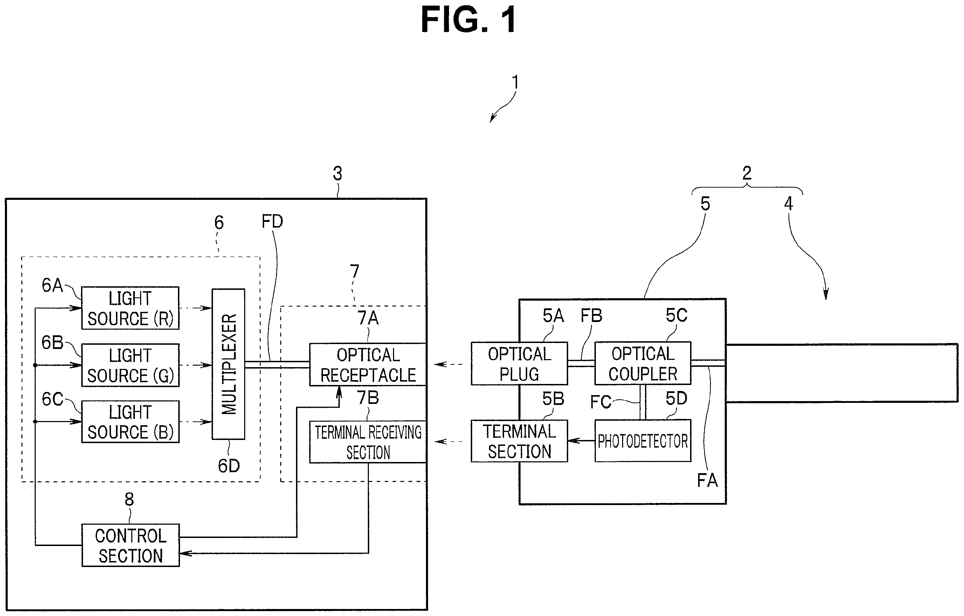

Fig. 1 to Fig. 9 relate to an embodiment of the present invention. Fig. 1 is a diagram illustrating a configuration of main parts of an endoscope system according to the embodiment.

An endoscope system 1 is configured by including an endoscope 2 and a main unit 3 as shown in Fig. 1.

The endoscope 2 is configured by including an insertion portion 4 configured by including an elongated shape so as to be inserted into a body cavity of a subject and a connector section 5 provided at a proximal end portion of the insertion portion 4 and configured to be attachable/detachable to/from a connector receiving section 7 (which will be described later) of the main unit 3.

Although not shown, an illumination optical fiber FA which is a single mode fiber extending from the connector section 5 is inserted into the insertion portion 4. An end on an emission end side of the illumination optical fiber FA, which is not shown, is disposed at a distal end portion of the insertion portion 4.

The connector section 5 is provided with an optical plug 5A and a terminal section 5B. An optical coupler 5C and a photodetector 5D are provided inside the connector section 5.

The optical plug 5A is configured to be attachable/detachable to/from an optical receptacle 7A (which will be described later) of the main unit 3. An end on an incident end side of an optical fiber FB which is a single mode fiber for transmitting light emitted through the optical receptacle 7A is disposed at the optical plug 5A.

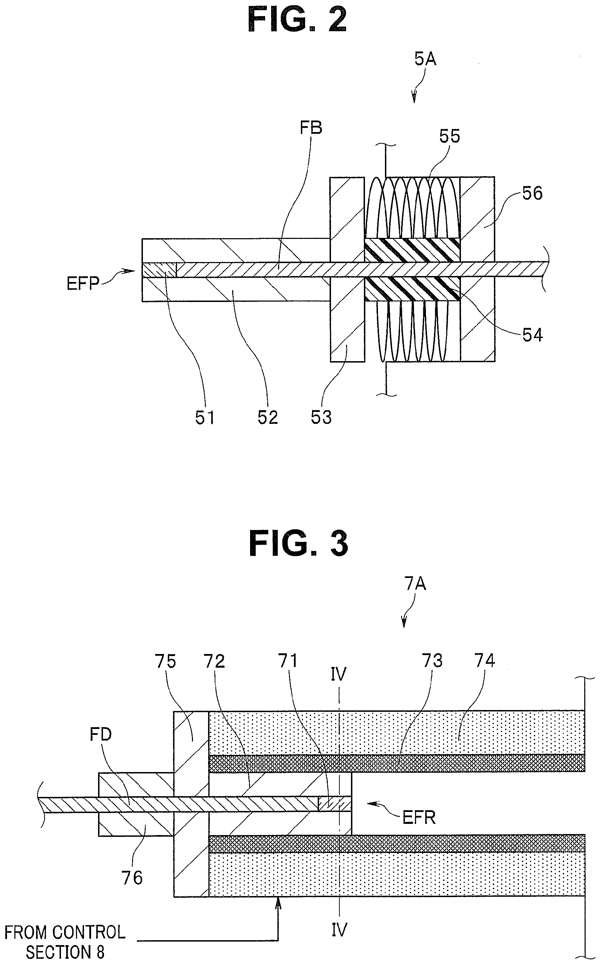

On the other hand, the optical plug 5A is configured by including a lens 51, a ferrule 52, a flange section 53, a covering member 54, a coil spring 55 and a holding member 56 as shown in, for example, Fig. 2. Fig. 2 is a diagram for describing an example of a configuration of the optical plug according to the embodiment.

A light emission surface of the lens 51 is disposed in proximity to an end face on an incident end side of the optical fiber FB. Furthermore, the lens 51 is formed of a GRIN rod lens provided with an optical characteristic (refractive index) capable of condensing, for example, light (parallel light) incident upon an incident surface after passing through the optical receptacle 7A (lens 71 which will be described later) and causing the light to enter an end face on the incident end side of the optical fiber FB.

The optical fiber FB is configured to transmit the illuminating light condensed by the lens 51.

The ferrule 52 is formed of, for example, zirconia or ceramic. The ferrule 52 is configured as a cylindrical body provided with a hole for holding (at least part of) the end on the incident end side of the optical fiber FB and the lens 51 along a central axis in a longitudinal direction.

An end face on one end side of the ferrule 52 in a longitudinal direction is provided so as to be exposed to an outside of the connector section 5. An end face on the other end side of the ferrule 52 in the longitudinal direction is bonded to a surface of a flange section 53.

That is, the optical plug 5A is configured by including a connection end face EFP (see Fig. 2) formed of the incident surface of the lens 51 and the end face on the one end side of the ferrule 52 in the longitudinal direction.

The flange section 53 is provided at a predetermined distance from a surface of the holding member 56 toward the outside of the connector section 5. A hole is provided in a central part of the flange section 53 to pass and position the optical fiber FB therethrough.

An end face on the other end side of the ferrule 52 in the longitudinal direction is bonded to the central part of the surface of the flange section 53. One end of the covering member 54 and one end of the coil spring 55 are respectively bonded to a back of the flange section 53.

That is, the flange section 53 is configured to hold the ferrule 52 in a way of cantilever.

The covering member 54 is formed of a member provided with elasticity such as resin. The covering member 54 is configured to have, for example, a cylindrical shape, cover a perimeter of the optical fiber FB in a space formed between the flange section 53 and the holding member 56 and expand or contract along the longitudinal direction of the optical fiber FB.

One end of the covering member 54 is bonded to the back of the flange section 53. The other end of the covering member 54 is bonded to the surface of the holding member 56.

The coil spring 55 is disposed so as to cover an outside of a side face of the covering member 54 in a space formed between the flange section 53 and the holding member 56 and is configured to expand or contract along the longitudinal direction of the optical fiber FB.

One end of the coil spring 55 is bonded to the back of the flange section 53. The other end of the coil spring 55 is bonded to the surface of the holding member 56.

The holding member 56 is fixedly disposed at a predetermined position inside the connector section 5. A hole is provided in a central part of the holding member 56 to pass and position the optical fiber FB therethrough.

The other end of the covering member 54 and the other end of the coil spring 55 are respectively bonded to the surface of the holding member 56.

The terminal section 5B is provided with one or more electric terminals formed of, for example, a metal piece and is configured to be attachable/detachable to/from the terminal receiving section 7B of the main unit 3.

An end face on an emission end side of the optical fiber FB, an end face on an incident end side of the illumination optical fiber FA and an end face on an incident end side of a branch optical fiber FC which is a single mode fiber connected to the photodetector 5D are disposed on the optical coupler 5C. The optical coupler 5C is configured to be able to branch light emitted from the end face on the emission end side of the optical fiber FB and cause the branched light to enter the end face on the incident end side of the illumination optical fiber FA and the end face on the incident end side of the branch optical fiber FC respectively.

The photodetector 5D is configured to detect a light quantity of light emitted from an end face on an emission end side of the branch optical fiber FC, generate an electric signal corresponding to the detected light quantity and output the electric signal to the terminal section 5B.

The main unit 3 is configured by including a light source section 6 configured to supply illuminating light to illuminate an object in the body cavity of a subject, a connector receiving section 7 configured to be attachable/detachable to/from the connector section 5 of the endoscope 2 and a control section 8.

The light source section 6 is configured by including a light source 6A, a light source 6B, a light source 6C and a multiplexer 6D.

The light source 6A is provided with a laser light source or the like that emits laser light having a red wavelength band (hereinafter abbreviated as "R light") and is configured to start emitting R light when it is turned on under the control of the control section 8 and stop emission of R light when it is turned off under the control of the control section 8.

The light source 6B is provided with a laser light source or the like that emits laser light having a green wavelength band (hereinafter abbreviated as "G light") and is configured to start emitting G light when it is turned on under the control of the control section 8 and stop emission of G light when it is turned off under the control of the control section 8.

The light source 6C is provided with a laser light source or the like that emits laser light having a blue wavelength band (hereinafter abbreviated as "B light") and is configured to start emitting B light when it is turned on under the control of the control section 8 and stop emission of B light when it is turned off under the control of the control section 8.

The multiplexer 6D is configured to be able to multiplex the R light emitted from the light source 6A, the G light emitted from the light source 6B and the B light emitted from the light source 6C and cause the multiplexed light to enter an end face on an incident end side of an optical fiber FD which is a single mode fiber.

That is, the light source section 6 is configured to be able to supply white light which is light provided with a plurality of wavelength bands (red color gamut, green color gamut and blue color gamut) to the optical fiber FD as illuminating light to illuminate an object in the body cavity of a subject.

The optical fiber FD is configured to transmit the illuminating light supplied from the light source section 6.

The connector receiving section 7 is provided with the optical receptacle 7A and a terminal receiving section 7B.

The optical receptacle 7A is configured to be attachable/detachable to/from the optical plug 5A of the endoscope 2. An end on an emission end side of the optical fiber FD is disposed on the optical receptacle 7A.

On the other hand, the optical receptacle 7A is configured by including, for example, a lens 71, a ferrule 72, a split sleeve 73, a piezoelectric element 74, a flange section 75 and a holding member 76 as shown in Fig. 3. Fig. 3 is a diagram for describing an example of the configuration of the optical receptacle according to the embodiment.

An incident surface of the lens 71 is disposed in proximity to the end face on the emission end side of the optical fiber FD. The lens 71 is formed of a GRIN rod lens provided with an optical characteristic (refractive index) capable of emitting light incident after passing through the optical fiber FD from an emission surface thereof as parallel light.

The ferrule 72 is formed of, for example, zirconia or ceramic. Furthermore, the ferrule 72 is configured as a cylindrical body provided with a hole for holding, for example, (at least part of) the end on the emission end side of the optical fiber FD and the lens 71 along a central axis in the longitudinal direction.

An end face on one end side of the ferrule 72 in the longitudinal direction is provided so as to be exposed in an inner space formed by the split sleeve 73. An end face on the other end side of the ferrule 72 in the longitudinal direction is bonded to a surface of the flange section 75.

That is, the optical receptacle 7A is configured by providing a connection end face EFR (see Fig. 3) formed of the light emission surface of the lens 71 and an end face on the one end side of the ferrule 72 in the longitudinal direction in the inner space formed by the split sleeve 73.

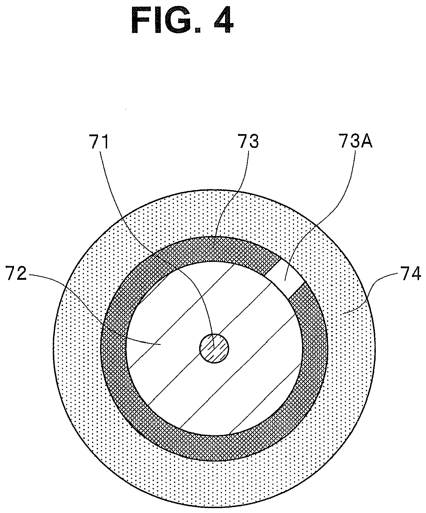

The split sleeve 73 is formed as a hollow cylindrical body provided with a slit 73A along the longitudinal direction as shown in, for example, Fig. 3 and Fig. 4. The split sleeve 73 is configured such that when the ferrule 52 of the optical plug 5A is inserted into the inner space, the split sleeve 73 is elastically deformed so as to be able to hold the ferrule 52 and the ferrule 72 with the connection end face EFP and the connection end face EFR being arranged opposite to each other via a gap while keeping the ferrule 52 and the ferrule 72 in a straight line. Fig. 4 is a cross-sectional view along a line IV-IV of Fig. 3.

One end of the split sleeve 73 in the longitudinal direction is provided so as to be exposed to the outside of the connector receiving section 7. On the other hand, the other end of the split sleeve 73 in the longitudinal direction is disposed in contact with a surface of the flange section 75.

The piezoelectric element 74 has a cylindrical shape as shown in, for example, Fig. 3 and Fig. 4 and is formed so as to cover a perimeter (whole circumference) of a side face of the split sleeve 73. Furthermore, the piezoelectric element 74 is configured so as to expand or contract (deform) along the longitudinal direction of the split sleeve 73 in accordance with a drive signal supplied from the control section 8.

Note that the piezoelectric element 74 of the present embodiment may not be formed so as to cover the perimeter of the side face of the split sleeve 73 as long as the piezoelectric element 74 is configured so as to expand or contract (deform) along the longitudinal direction of the split sleeve 73.

One end of the piezoelectric element 74 in the longitudinal direction is provided so as to be exposed to the outside of the connector receiving section 7. Furthermore, the other end of the piezoelectric element 74 in the longitudinal direction is bonded to the surface of the flange section 75.

A hole for passing and positioning the optical fiber FD therethrough is provided in a central part of the flange section 75.

An end face on the other end side of the ferrule 72 in the longitudinal direction is bonded to the central part of the surface of the flange section 75. Furthermore, the other end of the split sleeve 73 in the longitudinal direction is placed in contact with the outside of the bonded location of the ferrule 72 on the surface of the flange section 75. On the other hand, the other end of the piezoelectric element 74 in the longitudinal direction is bonded to the outside of the bonded location of the split sleeve 73 on the surface of the flange section 75. Furthermore, one end of the holding member 76 is bonded to a back of the flange section 75.

That is, the flange section 75 is configured to hold the ferrule 72 and the piezoelectric element 74 provided sandwiching part of the split sleeve 73 in a way of cantilever.

Note that according to the present embodiment, for example, when the piezoelectric element 74 is formed in a cylindrical shape, a member may be attached to the flange section 75 for expanding the surface area so as to match the thickness of the cylindrical shape.

The holding member 76 is fixedly disposed at a predetermined position inside the connector receiving section 7. A hole is provided in a central part of the holding member 76 to pass and position the optical fiber FD therethrough.

The terminal receiving section 7B is provided with one or more electric terminals formed of, for example, a metal piece and is configured to be attachable/detachable to/from the terminal section 5B of the endoscope 2.

That is, according to the above-described configuration, the optical fiber connector apparatus configured by including the optical plug 5A and the optical receptacle 7A is provided on a transmission path of illuminating light supplied from the light source section 6 to illuminate an object in the subject.

The control section 8 is provided with a CPU or the like and is configured to be able to perform control to turn ON/OFF the light sources 6A to 6C. Furthermore, the control section 8 is configured to be able to detect a state of connection between the connector section 5 and the connector receiving section 7 based on, for example, detection results of detecting a resistance value of the terminal receiving section 7B. Furthermore, upon detecting that the connector section 5 is connected to the connector receiving section 7, the control section 8 is configured to perform control (which will be described later) to adjust a gap between the connection end face EFP and the connection end face EFR based on an electric signal inputted via the terminal receiving section 7B, that is, according to a light quantity of illuminating light transmitted through the optical fiber FB after passing through the lens 51.

Note that though not shown, the endoscope system 1 of the present embodiment may also be configured so as to provide, for example, an image pickup section at a distal end portion of the insertion portion 4 to pick up an image of return light of illuminating light emitted to an object via the illumination optical fiber FA and output an image pickup signal and provide an image processing section in the main unit 3 to generate an observed image of the object based on the image pickup signal.

Furthermore, though not shown, the endoscope system 1 of the present embodiment may be configured so as to provide, for example, one or more piezoelectric elements at an end on the emission end side of the illumination optical fiber FA, provide a drive signal output section in the main unit 3 to output a drive signal for swaying the end so as to draw a predetermined track under the control of the control section 8 to the one or more piezoelectric elements, provide a light-receiving fiber in the insertion portion 4 to receive and guide return light of illuminating light emitted to the object according to the swaying of the end, provide a detector in the main unit 3 to detect the return light guided by the light-receiving fiber and output an electric signal and provide an image processing section in the main unit 3 to generate an observed image of the object based on the electric signal.

Next, operation of the endoscope system 1 according to the present embodiment will be described.

First, a user such as an operator turns on the power of the main unit 3 with the connector section 5 attached to the connector receiving section 7. Note that the following description will be provided, for simplicity of description, assuming that no drive signal is supplied to the piezoelectric element 74 for a period immediately after power to the main unit 3 is turned on until the control section 8 detects that the connector section 5 is connected to the connector receiving section 7.

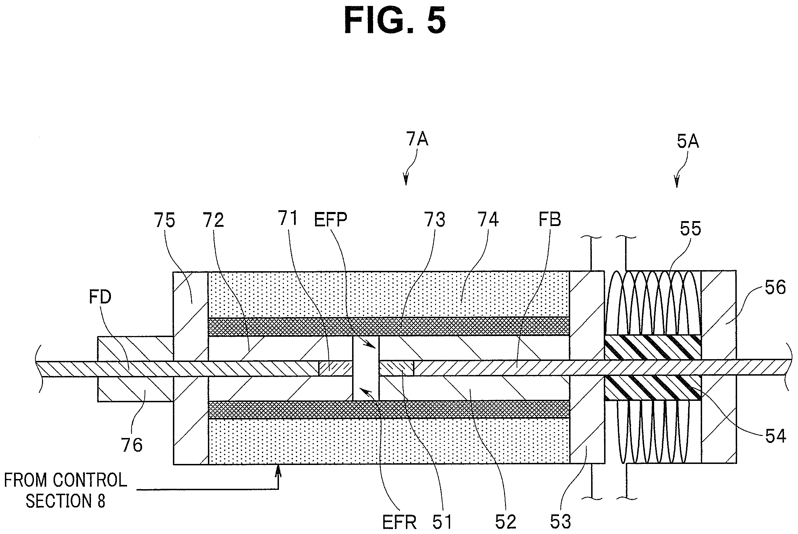

When the connector section 5 is attached to the connector receiving section 7, the optical plug 5A and the optical receptacle 7A are connected together and the terminal section 5B and the terminal receiving section 7B are electrically connected together. Furthermore, when the optical plug 5A and the optical receptacle 7A are connected together, for example, as shown in Fig. 5, and the connection end face EFP and the connection end face EFR are arranged opposite to each other via a gap in the inner space of the split sleeve 73. Furthermore, when the optical plug 5A and the optical receptacle 7A are connected together as shown in, for example, Fig. 5, one end of the split sleeve 73 in the longitudinal direction and one end of the piezoelectric element 74 in the longitudinal direction are pushed toward the flange section 75 side while remaining in contact with the surface of the flange section 53. Fig. 5 is a diagram illustrating an example of a state of connection between the optical plug and the optical receptacle according to the embodiment.

On the other hand, upon detecting that the power to the main unit 3 is turned on, the control section 8 performs control to turn on the light sources 6A to 6C from an off-state. Upon detecting that a resistance value of the terminal receiving section 7B abruptly decreases when the terminal section 5B is electrically connected to the terminal receiving section 7B, the control section 8 detects that the connector section 5 is connected to the connector receiving section 7. Upon detecting that the connector section 5 is connected to the connector receiving section 7, the control section 8 performs control to adjust a gap between the connection end face EFP and the connection end face EFR.

More specifically, while supplying a drive signal for causing the piezoelectric element 74 to expand or contract (deform) within a predetermined range along the longitudinal direction of the split sleeve 73, the control section 8 monitors a temporal variation of the signal level of an electric signal inputted via the terminal receiving section 7B.

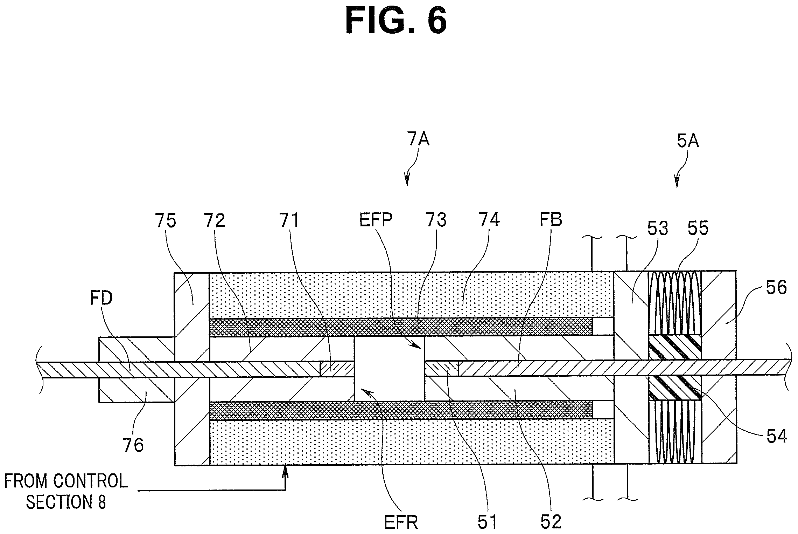

For example, in the case where the piezoelectric element 74 gradually expands from the state shown in Fig. 5 to a state shown in Fig. 6, a pressing force toward the flange section 53 side increases as the signal level of the drive signal supplied from the control section 8 to the piezoelectric element 74 gradually increases, the position of the flange section 53 displaces toward the holding member 56 side as the pressing force increases and the gap between the connection end face EFP and the connection end face EFR increases as the position of the flange section 53 displaces toward the holding member 56 side. Fig. 6 is a diagram illustrating an example different from that of Fig. 5 of the state of connection between the optical plug and the optical receptacle according to the embodiment.

For example, in the case where the piezoelectric element 74 gradually contracts from the state shown in Fig. 6 to the state shown in Fig. 5, a pressing force toward the flange section 53 side decreases as the signal level of the drive signal supplied from the control section 8 to the piezoelectric element 74 gradually decreases, the position of the flange section 53 displaces toward the flange section 75 side as the pressing force decreases and the gap between the connection end face EFP and the connection end face EFR decreases as the position of the flange section 53 displaces toward the flange section 75 side.

That is, the displacement mechanism of the present embodiment is provided with the flange section 53, the coil spring 55 and the piezoelectric element 74, and is configured such that the piezoelectric element 74 deforms along the longitudinal direction of the split sleeve 73 under the aforementioned control of the control section 8 so as to cause the connection end face EFP disposed in the inner space of the split sleeve 73 to displace. Furthermore, the spring mechanism of the present embodiment is provided with the flange section 53 and the coil spring 55, and is configured to expand or contract in accordance with the deformation of the piezoelectric element 74 when the optical plug 5A and the optical receptacle 7A are connected together to thereby cause the ferrule 52 to move forward or backward along the longitudinal direction of the optical fiber FB.

The control section 8 supplies a drive signal for causing the piezoelectric element 74 to expand or contract (deform) along the longitudinal direction of the sleeve 73 so that the signal level becomes the predetermined signal level SL based on the monitoring result of temporal variation of the signal level of the electric signal inputted via the terminal receiving section 7B.

Here, when, for example, coupling efficiency of R light is assumed to be ηR and coupling efficiency of G light is assumed to be ηG, the aforementioned predetermined signal level SL is set in advance as a signal level that satisfies a condition: ηB-Δη≤ηR≤B+Δη (Δη is a predetermined value greater than 0) and ηB-Δη≤ηG≤ηB+Δη for coupling efficiency ηB when the gap between the connection end face EFP and the connection end face EFR is optimized by B light.

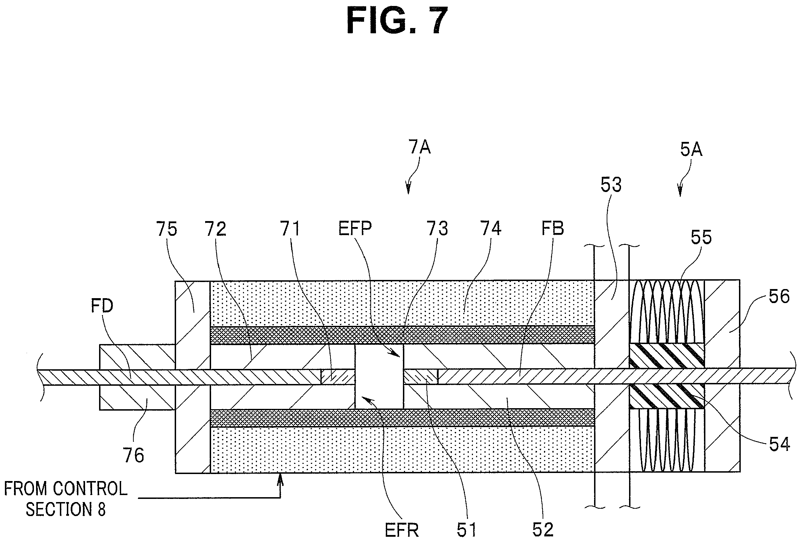

That is, according to the aforementioned control of the control section 8, when white light including R light, G light and B light is supplied from the optical receptacle 7A to the optical plug 5A, it is possible to adjust the gap between the connection end face EFP and the connection end face EFR so as to maintain a color balance between respective color components included in the white light as much as possible. According to such an adjustment of the gap, the optical plug 5A and the optical receptacle 7A are connected together in a state as shown in, for example, Fig. 7. Fig. 7 is a diagram illustrating an example different from those of Fig. 5 and Fig. 6 of the state of connection between the optical plug and the optical receptacle according to the embodiment.

Note that the control section 8 of the present embodiment continues to supply a drive signal for making the signal level of the electric signal inputted via the terminal receiving section 7B to the predetermined signal level SL until the control section 8 detects that the connector section 5 is removed from the connector receiving section 7 by detecting, for example, an abrupt increase of the resistance value of the terminal receiving section 7B.

As described above, according to the present embodiment, it is possible to change the gap between the connection end face EFP and the connection end face EFR every time the optical plug 5A and the optical receptacle 7A are connected together. For this reason, according to the present embodiment, it is possible to prevent the coupling efficiency from deteriorating as much as possible even when an object such as dust that may worsen the state of connection between the optical plug 5A and the optical receptacle 7A attaches to the surface of the flange section 53. That is, according to the present embodiment, it is possible to minimize fluctuations of coupling efficiency occurring in the joint for making contactless connections between optical fibers.

Note that the present embodiment is not limited to the case of adjusting the gap by changing the position of the connection end face EFP while fixing the position of the connection end face EFR, but may also be configured, for example, to adjust the gap by changing the position of the connection end face EFR while fixing the position of the connection end face EFP. More specifically, for example, when the optical receptacle 7A is provided with a member corresponding to the coil spring 55 instead of the piezoelectric element 74 and the optical plug 5A is provided with a member corresponding to the piezoelectric element 74 instead of the coil spring 55, the gap may be adjusted by changing the position of the connection end face EFR while fixing the position of the connection end face EFP.

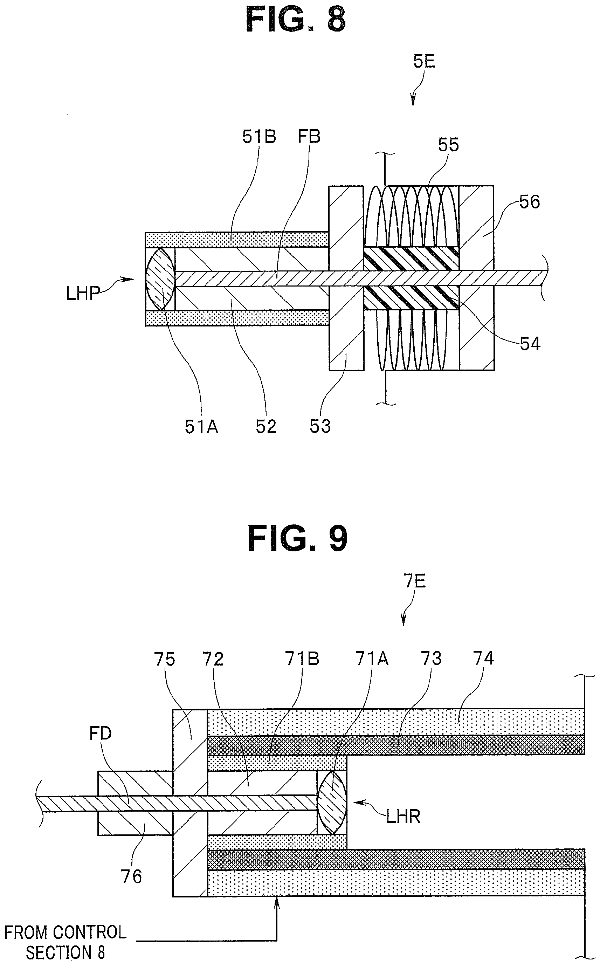

On the other hand, the optical fiber connector apparatus according to the present embodiment is not limited to the configuration including the optical plug 5A and the optical receptacle 7A, but may also be configured by including, for example, the optical plug 5E as shown in Fig. 8 and the optical receptacle 7E as shown in Fig. 9. Fig. 8 is a diagram for describing an example different from that of Fig. 2 of the configuration of the optical plug according to the embodiment. Fig. 9 is a diagram for describing an example different from that of Fig. 3 of the configuration of the optical receptacle according to the embodiment.

The optical plug 5E is configured by including a lens 51 A instead of the lens 51 in the optical plug 5A, and a housing 51B.

A light emission surface of the lens 51 A is disposed in proximity to the end face on the incident end side of the optical fiber FB. Furthermore, the lens 51A is formed of a convex lens provided with an optical characteristic (refractive index) capable of condensing light incident on an incident surface after passing through, for example, an optical receptacle 7E (lens 71 A which will be described later) and causing the light to enter the end face on the incident end side of the optical fiber FB.

The housing 51B is disposed at such a position as to cover an edge of the lens 51A and the perimeter of the side face of the ferrule 52. The housing 51B is configured to be positioned such that the optical axis of the lens 51A coincides with the central axis of the ferrule 52 in the longitudinal direction and have a cylindrical shape so as to be able to hold the lens 51A.

An end face on one end side of the housing 51B in the longitudinal direction is provided so as to be exposed to the outside of the connector section 5. Furthermore, an end face on the other end side of the housing 51B in the longitudinal direction is bonded to the surface of the flange section 53.

That is, the optical plug 5E is configured by including a connection end face LHP (see Fig. 8) including an incident surface of the lens 51A and an end face on one end side of the housing 51B in the longitudinal direction.

The optical receptacle 7E is configured by including a lens 71A instead of the lens 71 of the optical receptacle 7A and a housing 71 B.

An incident surface of the lens 71A is disposed in proximity to the end face on the emission end side of the optical fiber FD. Furthermore, the lens 71 A is formed of a convex lens configured to have an optical characteristic (refractive index) capable of condensing light incident after passing through the optical fiber FD and emitting the light from the light emission surface.

The housing 71 B is disposed at such a position as to cover an edge of the lens 71A and a perimeter of a side face of the ferrule 72. The housing 71B is configured to be positioned such that the optical axis of the lens 71A coincides with the central axis of the ferrule 72 in the longitudinal direction and have a cylindrical shape so as to be able to hold the lens 71 A.

An end face on one end side of the housing 71 B in the longitudinal direction is provided so as to be exposed to the inner space formed of the split sleeve 73. Furthermore, an end face on the other end side of the housing 71B in the longitudinal direction is bonded to the surface of the flange section 75. Furthermore, a side face of the housing 71B is covered with the split sleeve 73.

That is, the optical receptacle 7E is configured by providing a connection end face LHR (see Fig. 9) including a light emission surface of the lens 71 A and an end face on one end side of the housing 71B in the longitudinal direction in the inner space formed of the split sleeve 73.

Therefore, when the optical plug 5E and the optical receptacle 7E are used instead of the optical plug 5A and the optical receptacle 7A, it is also possible to prevent fluctuations of coupling efficiency for making contactless connections between optical fibers as much as possible.

The present invention is not limited to the aforementioned embodiment, but it goes without saying that various modifications and applications can be made without departing from the spirit and scope of the invention.

The present application claims a priority based on Japanese Patent Application No. 2014-027801, filed on February 17, 2014, the disclosure of which is incorporated in the specification of the present application, the scope of claims and the drawings by reference in its entirety.

## Claims

1. An optical fiber connector apparatus provided on a transmission path of light supplied from a light source section, the optical fiber connector apparatus comprising:
an optical receptacle comprising a first optical fiber configured to transmit light supplied from the light source section and a first lens disposed in proximity to an end face on an emission end side of the first optical fiber;
an optical plug comprising a second lens and a second optical fiber configured to transmit light condensed by the second lens;
a sleeve configured to hold the optical receptacle and the optical plug; and
a displacement mechanism configured to displace an opposite distance between the optical receptacle and the optical plug.

2. The optical fiber connector apparatus according to claim 1, wherein the optical receptacle further comprises a first ferrule configured to hold an end on an emission end side of the first optical fiber and the first lens, and
the optical plug further comprises a second ferrule configured to hold an end on an incident end side of the second optical fiber and the second lens.

3. The optical fiber connector apparatus according to claim 2,
wherein the sleeve is configured to hold the first ferrule and the second ferrule so that when the optical receptacle and the optical plug are connected together, a first connection end face formed of an emission surface of the first lens and an end face of the first ferrule and a second connection end face formed of an incident surface of the second lens and an end face of the second ferrule are arranged opposite to each other via a gap, and
the displacement mechanism is configured to displace either the first connection end face or the second connection end face disposed in an inner space of the sleeve by being controlled by a control section that performs control to adjust a gap between the first connection end face and the second connection end face in accordance with a light quantity of illuminating light transmitted by the second optical fiber after passing through the second lens when the optical receptacle and the optical plug are connected together.

4. The optical fiber connector apparatus according to claim 3, wherein the displacement mechanism is configured to comprise a piezoelectric element configured to deform along a longitudinal direction of the sleeve in accordance with the control by the control section.

5. The optical fiber connector apparatus according to claim 4, wherein the displacement mechanism further comprises a spring mechanism configured to expand or contract in accordance with the deformation of the piezoelectric element when the optical receptacle and the optical plug are connected together to thereby move the second ferrule forward or backward along the longitudinal direction of the second optical fiber.

6. The optical fiber connector apparatus according to claim 3, further comprising a photodetector configured to detect illuminating light transmitted by the second optical fiber after passing through the second lens when the optical receptacle and the optical plug are connected together and output an electric signal in accordance with a light quantity of the detected illuminating light,
wherein the piezoelectric element deforms in accordance with the control by the control section based on the electric signal so that a signal level of the electric signal becomes a predetermined signal level.

7. The optical fiber connector apparatus according to claim 1, wherein the light supplied from the light source section is light having a plurality of wavelength bands.

8. The optical fiber connector apparatus according to claim 1, wherein the first optical fiber and the second optical fiber are single mode fibers.

9. An endoscope system comprising an endoscope configured to comprise an insertion portion that can be inserted into a subject and a main unit connected to the endoscope and configured to comprise a light source section that supplies illuminating light for illuminating an object in the subject, the endoscope system comprising:
an optical receptacle provided in the main unit comprising a first optical fiber configured to transmit illuminating light supplied from the light source section and a first lens disposed in proximity to an end face of an emission end side of the first optical fiber;
an optical plug provided at a proximal end portion of the insertion portion comprising a second lens and a second optical fiber configured to transmit illuminating light condensed by the second lens;
a sleeve configured to hold the optical receptacle and the optical plug;
a control section configured to detect a state of connection between the endoscope and the main unit; and
a displacement mechanism configured to displace an opposite distance between the optical receptacle and the optical plug under the control of the control section.
